# EUROPEAN PATENT APPLICATION

(11) **EP 2 078 507 A1**
(43) Date of publication of application: **15.07.2009**
(21) Application number: 08000396.5
(22) Date of filing: 10.01.2008
(51) Int. Cl.: A61B 19/00, A61B 5/107

(54) **Small probing hook for arthroscopy**

(71) Applicant: Ludwig-Maximilians-Universität München, 80359 München (DE)
(72) Inventor: Jansson, Volkmar, Prof. Dr. Dipl.-Ing., 82205 Gilching-Geisenbrunn (DE); Müller, Peter, Prof. Dr., 86899 Landsberg am Lech (DE); Feist, Markus, Dr., 81667 München (DE); Markwitz, Robert, Dipl.-Ing., 80469 München (DE)
(74) Representative: Vossius & Partner

(57) **Abstract**

The present invention relates to an arthroscopic probe with an elongated shaft. The shaft comprises a distal and a proximal end and a bore extending at least substantially parallel to said shaft with an opening of said bore at the distal end of said shaft. The arthroscopic probe further comprises a longitudinal probe with a distal end which is flexible, wherein the longitudinal probe is arranged in said bore and is longitudinally displaceable in said bore and displaceable to extend from said opening. The arthroscopic probe also comprises a means for indicating a length of the extended longitudinal probe.

## Description

### FIELD OF THE INVENTION

The present invention relates to a probing hook for arthroscopic use. In particular, the present invention relates to a small probing hook with a flexible longitudinal probe which is displaceable for measuring the size of cartilage defects with higher accuracy.

### BACKGROUND OF THE INVENTION

Minimally invasive surgery, like arthroscopic surgery constitutes a benefit to patients because of smaller incisions, less pain, less trauma and shorter healing periods, but the surgeon must cope with loss of direct tactile information and reduced visual information. In particular, the surgeon must operate through a narrow tube, which is complicated. Typically, only one probe can be used at a time. Often the viewing camera is positioned at an angle different from the surgeon's normal gaze. This contrasts to "open surgery" where the surgeon may easily view the surgical site and can freely move both hands.

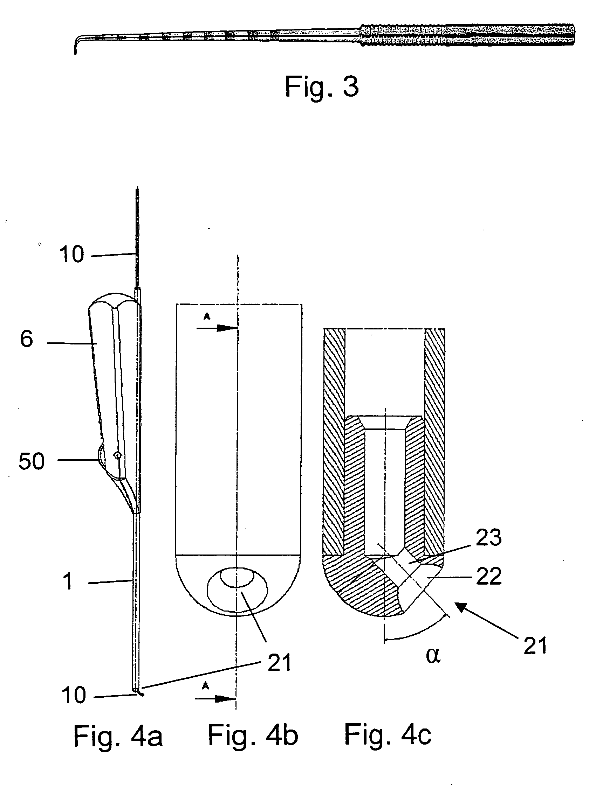

During arthroscopy, a surgeon inserts a small camera into one incision and a surgical means/instrument/device or an arthroscopic probe into another. One common arthroscopic probe, often also called arthroscopic hook or arthroscopic small probing hook, is a simple device with a shaft having a metal tip at one end of the shaft and a handle at the other end of the shaft (see Fig. 3). A plurality of small probing hooks is known with many different tip shapes, but the "arthroscopic hook" with a tip bent to a 90° angle is commonly used. The bent tip permits probing of the surface of tissues, including ligaments, menisci and cartilage, to find anomalies. Typically, the tip of an arthroscopic hook further comprises a graduation. The graduation on the tip of the probe is helpful to estimate the size of possible cartilage defects.

Depending on the nature and size of cartilage defects (surface lesion) different therapeutic methods are usually preferred. The defect size and the corresponding preferred therapy can be classified in three classes: (i) micro fracturing < 2 cm²; (ii) OATS (Osteochondral Autograft Transfer System) 2 - 4 cm²; (iii) ACT (Autologous Chondrocyte Transplantation) > 4 cm². Therefore, a reproducible and valid measurement of the defect size during arthroscopy is essential.

During arthroscopy, a tiny beam of light is shining into the joint through a set of optic fibres attached to a series of rod lenses that magnify the image as well as direct the image typically at 25° to 30° away from the direction of the scope itself. All of this is contained within a narrow gauge tubular instrument about 5 mm in diameter. The advantage of these angulations of 25° to 30° is, of course, the fact that by rotating the scope the surgeon can see a much wider field of vision without moving the telescope from side to side. The disadvantage, however, is the distorted viewing which makes it difficult to estimate the real size of the structures.

In estimating the size of certain structures, e.g. a cartilage defect, the graduation on the tip of a conventional arthroscopic hook serves as an object of comparison. In particular, the tip of the arthroscopic hook with its graduation is typically placed in front of a cartilage defect to estimate the size of the cartilage defect on the basis of the graduation. Typically, photos are taken during such a measurement for documentation and later analysis. However, since the shaft with the tip of such a probe is rigid, it is principally impossible to place the shaft with the graduation parallel or planar to the defected surface. Thus, due to the angulation of the optics and the angulation of the arthroscopic hook, the estimation of the defect size during arthroscopy tends to comprise large faults and imperfections. Based on such potentially erroneous estimations of the defect size a wrong therapy might be chosen.

The use of computer aided navigation systems, e.g., OrthoPilot® by B|Braun/Aesculap, a tool for the precise execution of surgical interventions, is helpful for a more precise measurement of defects. However, such systems are complicated and cost-intensive and often too sophisticated for easy diagnostics.

It is therefore an object of the present invention to provide an arthroscopic instrument for (clinical) diagnostics which allows a reproducible and more precise measurement of the cartilaginous defect size during arthroscopy.

### SUMMARY OF THE INVENTION

This object is achieved by the features of the independent claim. Further preferred embodiments are exemplified in the dependent claims.

An arthroscopic probe according to a first aspect of the invention comprises an elongated shaft with a distal and a proximal end, said shaft having a bore extending at least substantially parallel to said shaft with an opening of said bore at the distal end of said shaft. Preferably, the arthroscopic probe further comprises a longitudinal probe with a distal end, which is flexible. The longitudinal probe may be arranged in said bore and being longitudinally displaceable in said bore and displaceable to extend from said opening; and means indicating a length of the extended longitudinal probe. In other words, the bore is adapted to receive a longitudinal probe which is at least flexible at the distal end. Preferably, the longitudinal probe provides flexibility along a substantial part of the probe, preferably the entire longitudinal probe provides the desired flexibility. The longitudinal probe may also comprise zones or sections with different flexibility. For instance, the longitudinal probe provides a higher flexibility at the distal end and a lower flexibility at the proximal end.

In case the longitudinal probe is not located within the bore of the arthroscopic probe, the distal opening of the bore may be closed with a mandrel (often also called mandrin or mandril). The mandrel in the opening prevents a contamination of the opening and/or the bore.

An advantage of the invention is the particularly easy handling. Preferably, the size and/or the design of the arthroscopic probe according to the present invention is similar to conventional arthroscopic probes. It is a further advantage that the arthroscopic probe according to the present invention offers a user the same diagnostic capability as the known instruments, without any exchange of instruments for measuring possible defects in articular joint surfaces with higher accuracy.

A shaft with a longitudinal bore preferably houses the flexible longitudinal probe. The arthroscopic probe preferably comprises a means for the displacement of the flexible longitudinal probe inside the bore so that it may be extended from the distal end of the bore, i.e., the opening, for measuring the size of, e.g., cartilage defects (surface lesions) during arthroscopy. In case the flexible longitudinal probe is withdrawn, i.e. the probe is substantially completely located within the bore and does not extend from the distal end of the shaft, the arthroscopic probe permits mechanical probing of the structure and the examination of the joint. Thus, the arthroscopic probe according to the present invention has the advantage that an experienced surgeon can use the arthroscopic probe similar like commonly known probing hooks, i.e. the surgeon does not have to relearn the use of the arthroscopic probe. In other words, the arthroscopic probe according to the present invention provides the same functionality as a standard probing hook in case the flexible longitudinal probe is retracted inside the bore.

However, the extracted flexible longitudinal probe allows a much more accurate measurement of the size of defects since the flexible longitudinal probe may be placed substantially parallel or planar to the surface with the defect, i.e. uncertainties due to the angular relationship between the probing hook, the optics and the surface with the defect can be avoided. In other words, the planar placement of the flexible longitudinal probe avoids optical distortions.

The arthroscopic probe according to the present invention preferably provides at least at the distal end of the longitudinal probe a flexibility which allows it to be articulated but has a self-holding effect at least over an operational length of the extended longitudinal probe. The flexible longitudinal probe may also provide the desired flexibility over the total length of the probe. In other words, the longitudinal probe is preferably flexible enough that it can be bent easily without breakage of the longitudinal probe. However, the longitudinal probe is firm enough to provide a self-holding effect, i.e. the longitudinal probe can be placed inside a joint substantially parallel to the joint surface with the defect. For instance, a typical yarn provides flexibility but not the required self-holding effect. On the other hand, an optical fiber material may provide flexibility but not the required self-holding effect. Preferably, the flexible longitudinal probe is able to undergo repeated high strain deformations without plastic deformation. The longitudinal probe is preferably a flexible element, like a filament, a wire or a (micro) spiral spring.

According to yet a further preferred embodiment, the entire probe or at least the distal end of the probe may be provided with a bias bend or initial tension. Such a bias bending of the probe may further allow that the extended distal end of the longitudinal probe may be placed substantially parallel to the articular surface with the defect. In other words, the initial tension of the probe provides a bias force to the longitudinal probe which ensures that the extended distal end abuts to the defect of the articular surface.

The arthroscopic probe may further comprise a handle at the proximal end of the elongated shaft. The handle gives a firm grip and aids in instrument control. The arthroscopic probe preferably comprises only material(s) which allow that the probe may be disinfected, steam autoclaved or ethylene oxide sterilized. Preferably, the shaft, the handle and the tip are integrally formed (formed from one piece). The arthroscopic probe may be delivered/sold with or without the longitudinal probe. Irrespectively, whether the arthroscopic probe is delivered/sold with or without the longitudinal probe, it is preferred that the longitudinal probe may be removed from the shaft and the handle such that a separate cleaning and/or disinfection of the arthroscopic probe and the longitudinal probe is possible. According to a further aspect, the longitudinal probe may be disposable.

According to a further aspect, a mandrel may close the distal and/or the proximal opening of the bore. According to a further aspect, the mandrel may close the opening(s) when the longitudinal probe is located with in the bore. According to an other aspect, the mandrel may close the opening(s) when the longitudinal probe is not located with in the bore. The mandrel may be cleaned and/or disinfected separately from the shaft and the longitudinal probe. According to a further aspect, the mandrel may be disposable.

In order to use the arthroscopic probe for mechanical probing of the joint, the elongated shaft is substantially rigid. Preferably, the shaft is hardened to resist breakage. The shaft may be tapered, preferably with a larger cross section at the proximal end and a smaller cross section at the distal end.

Preferably, the bore of the arthroscopic probe extends axially through the shaft. In case the arthroscopic probe comprises a handle, the bore preferably further extends through the handle in the distal-proximal direction.

The arthroscopic probe according to the present invention may be further designed such that the distal end of the shaft together with the bore are bent in an angle relative to the longitudinal axis of the elongated shaft, preferably in a range between 45° to 90° to form a small hook at the distal end. However, the bending angle of the bore and the shaft may differ. For example, the shaft may be bent by 90°, whereas the bore may be bent only 45° such that the opening of the bore is not at the distal end of the shaft but slightly displaced. Preferably, the hook comprises a predetermined shape, wherein the material of the shaft and/or the hook is rigid enough such that the small hook will not be deformed when probing a joint, i.e. the hook and the shaft will not be deformed when typical probing forces are applied to the probe.

According to another aspect of the present invention, the distal end of the shaft together with the bore are bendable in an angular range between 45° to 90° relative to the longitudinal axis of the elongated shaft. According to this aspect of the invention, the material of the shaft and/or the hook is as well rigid enough such that the small hook will not be deformed when probing a joint, however, the hook may be deformed within a predetermined angular range when heat or an electric current is applied to the distal end of the probe. For instance, at least the distal end of the shaft may be made from a shape memory alloy (e.g. copper-zinc-aluminium-nickel, copper-aluminium-nickel, and nickel-titanium (NiTi)) such that the hook at the distal end may be bendable within the predetermined angular range by applying an electric current and/or heat. For energising the shape memory alloy, the arthroscopic probe according to the present invention may further comprise a battery to allow a change of the form of the distal end of the shaft.

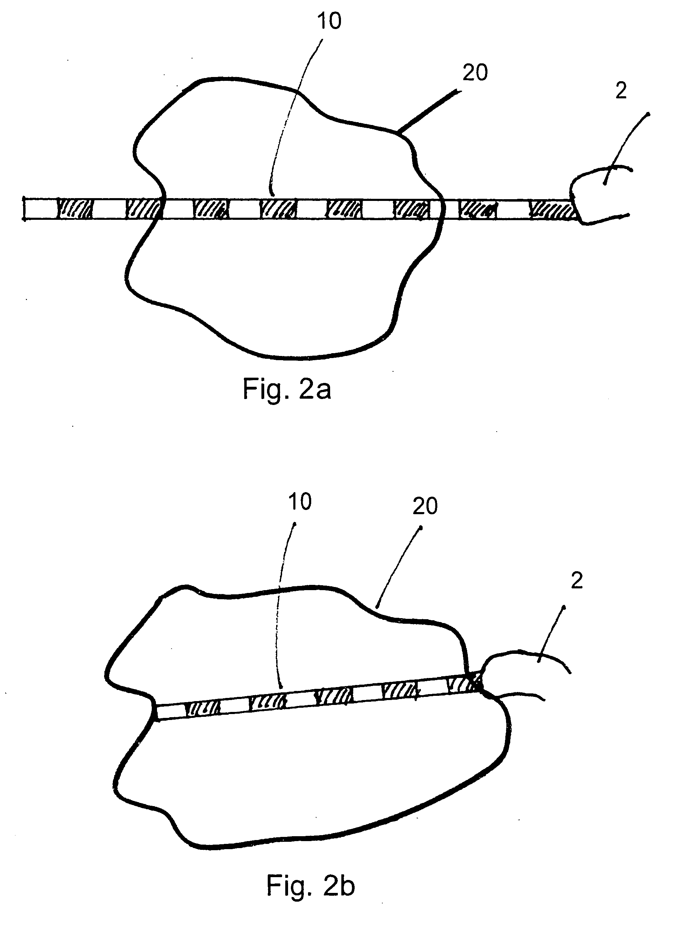

A further advantage of the present invention is provided by the means for indicating a length of the extended longitudinal probe. The means for indicating a length may be realized by marks and/or a scale on the flexible longitudinal probe, preferably at least on the distal end of the extended longitudinal probe. Such marks or scale provide the advantage that the defect of the joint can be easily measured when the flexible longitudinal probe is placed in front of or parallel to the articular surface with the defect. A surgeon measures the defect size (e.g. the length) by simply comparing the scale with the defect, like done in normal length measurement with a ruler. This kind of measurement provides the further advantage that said "comparison" (measurement) can be easily documented by a photo. The total extracted length (also called "operational length") of the extracted longitudinal probe is substantially irrelevant for this kind of measurement, as long as the total extracted length is at least longer than the defect size to measure, since the size of the defect is visually compared with the longitudinal probe inside the joint (see, e.g., Fig. 2a). In other words, the defect is visually compared with the reference scale on the flexible longitudinal probe. It is not necessary that the distal end of the extracted longitudinal probe be aligned with the edge of the defect since the measurement is a relative measurement.

According to a further aspect of the invention the shaft and/or the handle may comprise an inspection window for inspecting the marks and/or the scale provided on the longitudinal probe for measuring the extracted length of the longitudinal probe. For this kind of size measurement, it is preferred that the distal end of the flexible longitudinal probe is aligned with a first edge of the defect and the distal end of the shaft (tip) is aligned with a second edge of the defect (see, e.g., Fig. 2b). Thus, the distance between the first edge and the second edge corresponds to the total length of the extracted part of the longitudinal probe. This length may be measured visually within the joint through the optics and/or documented by a photo, similar to the above-mentioned measurement. However, the inspection window for inspecting the marks and/or the scale provides a further opportunity to measure the length of the extracted part of the longitudinal probe.

Preferably, the arthroscopic probe according to the present invention comprises an operating device, in particular in the form of a sliding key, for the longitudinal displacing of said longitudinal probe within said bore. Such a sliding key is preferably connected or affixed, e.g. by clamping, adhesive, etc., to the longitudinal probe. By means of the sliding key, longitudinal displacement of the longitudinal probe from the "probing position" (withdrawn position of the flexible longitudinal probe) to the "measuring position" (extracted position of the flexible longitudinal probe) can be achieved without putting down the instrument. The position of the sliding key, which is preferably within an easy gripping range, permits the use of the probing hook for left-handed as well as for right-handed persons. Moreover, the actual position of the sliding key also allows a measurement of the length of the extracted part of the longitudinal probe, in case the sliding key is movable relative to markings or a scale. In other words, the arthroscopic probe according to the present invention may comprise means for indicating a length of the extended longitudinal probe in the form of marks and/or a scale on the shaft and/or handle for measuring a displacement of the sliding key.

According to yet a further aspect of the present invention, the arthroscopic probe may comprise an operating device for displacing the longitudinal probe, in the form of a wheel, preferably in frictional engagement with the longitudinal probe. Thus, instead of moving a sliding key, which is affixed to the longitudinal probe, along the shaft or the handle for the displacement of the longitudinal probe, a wheel may be provided at the handle or the shaft. In case the wheel is in frictional engagement with the longitudinal probe, a rotation of the wheel will result in a longitudinal displacement of the longitudinal probe within the bore. A person skilled in the art will appreciate that any other kind of engagement may be used which results in a longitudinal displacement of the longitudinal probe by rotational movement of the wheel, e.g. gearing, etc.

The opening at the distal end of the arthroscopic probe according to the present invention may be closable by a mandrel. The mandrel may be an extra part, or may be part of the longitudinal probe. For instance, the distal end of the longitudinal probe may comprise a slightly larger diameter at a predetermined length. The slightly larger diameter is preferably adjusted to the diameter of the opening of the distal end of the shaft such that there exists a tight fitting between the longitudinal probe and the opening. This ensures that external particles will not enter into the opening when the longitudinal probe is in the retracted (withdrawn or probing) position. On the other hand, the slightly smaller diameter of the rest of the longitudinal probe reduces the friction between the longitudinal probe and the bore such that the force for displacing the longitudinal probe is reduced.

Other objects, advantages and novel features of the present invention will become apparent from the following detailed description of the invention when considered in conjunction with the accompanying drawings.

### BRIEF DESCRIPTION OF THE DRAWINGS

Figure 1 schematically shows a cross sectional view of a preferred embodiment of the arthroscopic probe according to the present invention.
Figures 2a and 2b schematically show the measurement of a defect with the scale on the longitudinal probe.
Figure 3 shows a prior art arthroscopic hook probe.
Figures 4a-4c show a further preferred embodiment of the arthroscopic probe according to the present invention.
Figures 5a-5f show different views of a further preferred embodiment of the arthroscopic probe according to the present invention with an operating device in the form of a wheel.
Figures 6a-6c show different views of a further preferred embodiment of the arthroscopic probe according to the present invention with a means for indicating the length of the extended longitudinal probe in the form of a wheel.
Figures 7 and 8 show different perspective views of the embodiment of Fig. 6.
Figure 9 shows an extracted longitudinal probe at a distal end of a shaft, wherein the shaft is not bent at the distal end.
Figure 10 shows a simulated usage of an arthroscopic probe at an artificial knee joint.

### DETAILED DESCRIPTION OF THE EMBODIMENTS

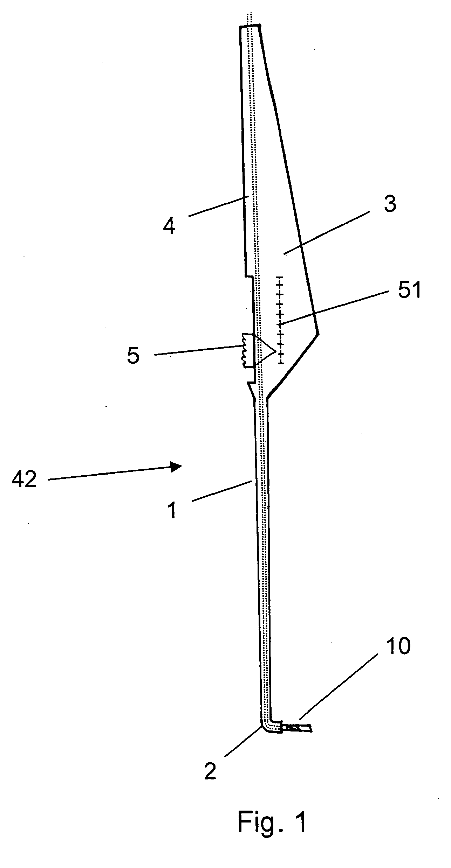

Referring to Fig. 1, a small probing hook 42 according to the present invention comprises a handle 3 in the shape of a gripping handle and a shaft 1 with a diameter of approximately 2-4 mm connected to the handle 3. According to the embodiment depicted in Fig. 1, the distal end 2 of the shaft 1 is bent by approximately 90°. A possible bending radius is in the range between 2 to 5 mm so that it may be used during diagnostic procedures to examine parts of a joint by feeling and pulling. The probing hook 42 is equipped with a flexible longitudinal probe (flexible element) 10 which is displaceably arranged in a bore 4 extending through the handle 3 and the shaft 1.

At the distal end of the shaft, the bore is curved in such a manner that the flexible longitudinal probe emerges from the opening at the distal end 2. However, the bore may comprise a different bending angle such that the longitudinal probe be sloped at an angle of less than the bending angle, e.g. less than 90° with respect to the shaft axis. Depending on the use of the probing hook other angles for the bent distal end of the shaft may be further preferred, such as 10°, 20°, 30°, 40°, 50°, 60°, 70°, 80°, 90° or even more.

The displacement of the flexible longitudinal probe 10 is aided by means of the operating means in the form of a sliding key 5. In the embodiment shown, the sliding key 5 is clamp-connected with the flexible longitudinal probe 10. The sliding key 5 allows an easy displacement of the flexible longitudinal probe 10 from the probing position to the measuring position without putting down the instrument. The position of the sliding key, which is within an easy gripping range, permits the use of the probing hook for left-handed as well as for right-handed persons.

The longitudinal displacement of the flexible element provides the advantage that during a probing application, the flexible longitudinal probe can be completely withdrawn into the bore to avoid impairment of the patient by the flexible longitudinal probe. In other words, in case the flexible longitudinal probe is retracted in the bore, i.e. does not extend through the opening of the bore, the probing hook may be used by a surgeon like a conventional probing hook, which is used for feeling and pulling structures within the knee joint. Since the first part of any arthroscopy should involve only the arthroscope and the probing hook, an arthroscope and probing hook are all that are needed to perform a professional diagnostic arthroscopy.

Since, during a diagnostic procedure, the shaft 1 is acted upon by considerable bending forces, it is preferred that the shaft is substantially rigid or rigid. To enhance the rigidity of the shaft, the diameter of the shaft 1 may be enlarged in the proximal direction, e.g. the shaft comprises a tapered or conical shape with a larger diameter in the proximal end of the shaft and a smaller diameter in the distal end of the shaft.

During diagnostic procedure, the opening at the distal end may be closed with a mandrel (not shown) which further avoids contamination of the opening and/or the bore.

The actual position of the sliding key 5 also allows a measurement of the length of the extracted part of the longitudinal probe 10, in case the sliding key is movable relative to a marking or a scale 51. In other words, the arthroscopic probe according to the present invention may comprise a scale 51 on the handle 3 indicating a length of the extended longitudinal probe.

The flexible longitudinal probe 10 comprises marks or a scale such that the defects may be measured easily. Two different measurement methods will be discussed with regard to Figs. 2a and 2b. In particular, Figure 2a shows the method by which a surgeon measures the defect size (e.g., the length) by simply comparing the scale on the longitudinal probe 10 with the defect 20. The flexibility of the longitudinal probe allows a planar placement of the longitudinal probe to the surface with the defect. Such a "comparison" (measurement) can be easily documented by a photo without any optical distortions. The total extracted length (also called "operational length") of the extracted longitudinal probe is substantially irrelevant for this kind of measurement, as long as the total extracted length is at least longer than the defect size to measure, since the size of the defect is visually compared with the longitudinal probe inside the joint.

The measured displacement of the sliding key 5 by the scale 51 provides a further measurement method. For this kind of size measurement, it is preferred that the distal end of the flexible longitudinal probe is aligned with a first edge of the defect 20 (e.g., the left side in Fig. 2b) and the distal end of the shaft 2 is aligned with a second edge of the defect (e.g., right side of the defect in Fig. 2b). The distance between the first edge and the second edge of the defect corresponds to the total length of the extracted part of the longitudinal probe. This length may additionally or alternatively be measured visually within the joint through the optics and/or documented by a photo, similar to the above-mentioned measurement.

Figure 3 shows a conventional probing hook with markings on the shaft.

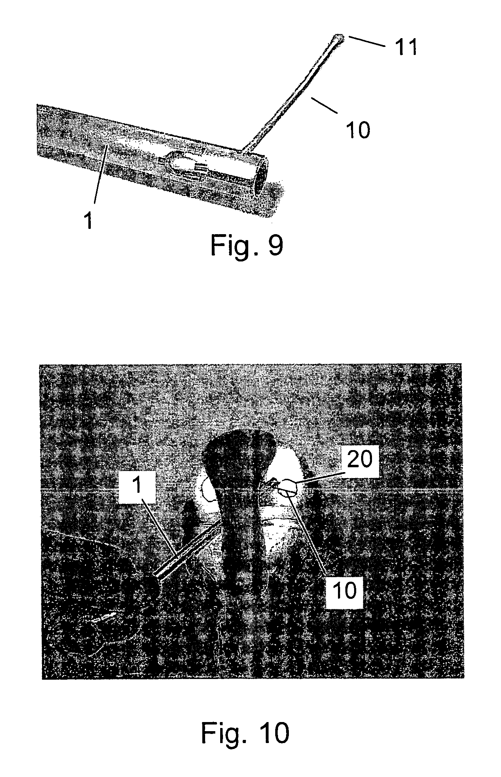

Figure 4a shows a further embodiment of the present invention in a perspective view. A casing 6 is mounted to the shaft 1. The casing 6 supports a wheel 50 which is in frictional engagement with the longitudinal probe 10. The shaft 1 is not bent at the distal end but comprises an opening 21 displaced from the longitudinal axis A of the shaft. Figure 4b shows the distal end of the shaft 1 enlarged with the opening 21. In particular, the centre axis of the opening 21 is displaced from the longitudinal axis A by the angle α = 45° as can be seen in the cross sectional view of the distal end of the shaft in Figure 4c. Moreover, the cross sectional view of Figure 4c reveals that the opening 21 comprises two different diameters, a larger diameter 22 and a smaller diameter 23. The smaller diameter 23 of the opening 21 is preferably the same as the diameter of the longitudinal probe. The longitudinal probe may further comprise an enlarged distal end 11 (see, e.g., Fig. 9), wherein the enlarged distal end 11 comprises a diameter similar to the larger diameter 22 of the opening 21. Thus, in case the longitudinal probe is in the retracted position (probing position), the opening and the bore are closed by the enlarged distal end 11 such that external particles will not enter the opening 21. According to another embodiment, the opening 21 may be closed by an additional mandrel (not shown).

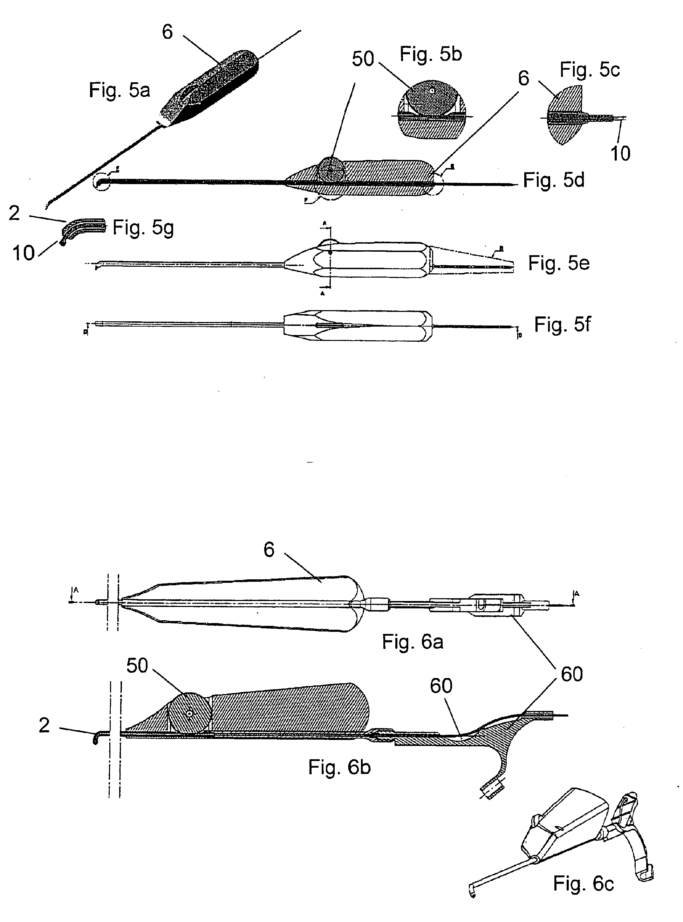

Figures 5a to 5g show a further preferred embodiment of the present invention. In contrast to Figure 4a, the shaft is bent at the distal end, which is shown in more detail in the cross sectional view of Fig. 5g. Figure 5b shows a detailed cross sectional view of the mechanism of the operating device in the form of a wheel 50. As can be seen in Figure 5b, the wheel is in frictional engagement with the longitudinal probe such that a rotational movement of the wheel 50 results in a longitudinal movement of the longitudinal probe 10. Figure 5c shows an enlarged cross sectional view of the proximal end of the casing 6. Figure 5d shows the entire arthroscopic probe in a cross sectional view; Figure 5e shows the probe in a side view and Figure 5f shows the probe in a top view.

Figures 6a, 6b and 6c show a further preferred embodiment, wherein the arthroscopic probe comprises a casing 6 with a wheel 50 similar to the embodiment of Figures 5a to 5g. However, the arthroscopic probe with the casing 6 and the wheel may be sold without the longitudinal probe. Preferably, the distal opening of the bore 4 is closed with a mandrel in order to avoid a contamination of the bore during probing of the joint. After probing, an additional grip 60 may be mounted to the proximal end of the arthroscopic probe. In particular, the additional grip 60 may be mounted to the arthroscopic probe such that a longitudinal flexible probe is provided by the grip 60 and inserted into the bore 4 at the proximal end. The longitudinal probe may be advanced within the bore 4 by pushing the longitudinal probe 10 with a finger within the concave portion 61 of the grip 60. Figure 6a is a top view, Figure 6b is a cross sectional view and Figure 6c is a perspective view.

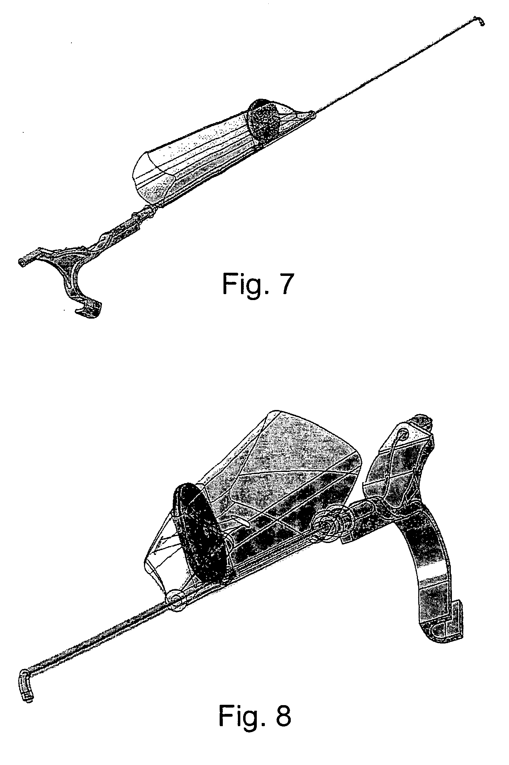

Figures 7 and 8 show the same embodiment as depicted in Figures 6a to 6c but in different perspective views.

Figure 9 shows an enlarged view (photo) of the distal end of the shaft 1 with an extracted longitudinal probe 10. As can be seen in this Figure, the longitudinal probe comprises an enlarged tip 11. The enlarged tip in this embodiment has the form of a pinhead.

Finally, Figure 10 shows a simulated usage of an arthroscopic probe at an artificial knee joint. As can be seen in Figure 10, the shaft 1 of the arthroscopic probe is guided under the "ligamentum patellae". The surgeon can measure the size of the defect 20 with the extracted longitudinal probe 10.

Preferred embodiments of the invention have been described in considerable detail. Many modifications and variations to the embodiments described will be apparent to those skilled in the art. In particular, features alone or in different combinations as in the specifc embodiment described above are also within the scope of the present invention. Therefore, the invention should not be limited to the embodiments described, but should be defined by the claims that follow.

## Claims

1. An arthroscopic probe with:
an elongated shaft with a distal and a proximal end,
said shaft having a bore extending at least substantially parallel to said shaft with an opening of said bore at the distal end of said shaft;
a longitudinal probe with a distal end, which is flexible, the longitudinal probe being arranged in said bore and being longitudinally displaceable in said bore and displaceable to extend from said opening;
means indicating a length of the extended longitudinal probe.

2. The arthroscopic probe according to claim 1, wherein the distal end of the longitudinal probe has flexibility which allows it to be articulated but has a self-holding effect at least over an operational length of the extended longitudinal probe.

3. The arthroscopic probe according to claim 1 or 2, further comprising a handle, wherein said proximal end of the elongated shaft is connected to said handle.

4. The arthroscopic probe according to claim 1, 2 or 3, wherein said elongated shaft is substantially rigid.

5. The arthroscopic probe according to any of the preceding claims, wherein said bore is axially extending through said shaft.

6. The arthroscopic probe according to any of the preceding claims, wherein said bore is further extending through said handle.

7. The arthroscopic probe according to any of the preceding claims, wherein said longitudinal probe is a flexible element, like a filament, a wire or a (micro) spiral spring.

8. The arthroscopic probe according to any of the preceding claims, wherein the distal end of said shaft together with said bore are bent in an angle relative to the longitudinal axis of the elongated shaft in a range between 45° to 90° to form a small hook at the distal end.

9. The arthroscopic probe according to any of the preceding claims, wherein the distal end of said shaft together with said bore are bendable in an angle relative to the longitudinal axis of the elongated shaft in a range between 45° to 90° to form a small hook at the distal end.

10. The arthroscopic probe according to any of the preceding claims, wherein said means for indicating a length of the extended longitudinal probe are marks and/or a scale on the longitudinal probe, preferably at least on the distal end of the extended longitudinal probe.

11. The arthroscopic probe according to claim 10, wherein the shaft and/or the handle comprises an inspection window for inspecting the marks and/or the scale provided on the longitudinal probe for measuring displacement of the longitudinal probe.

12. The arthroscopic probe according to any of the preceding claims, further comprising an operating device, in particular in the form of a sliding key, for longitudinally displacing said longitudinal probe in said bore.

13. The arthroscopic probe according to claim 12, wherein the means indicating a length of the extended longitudinal probe are marks and/or a scale on the shaft and/or a handle for measuring a displacement of the sliding key.

14. The arthroscopic probe according to claim 12, wherein the operating device for displacing the longitudinal probe comprises a wheel, preferably in frictional engagement with the longitudinal probe.

15. The arthroscopic probe according to any of the preceding claims, wherein the opening is closable by a mandrel.

16. The arthroscopic probe according to any of the preceding claims, wherein at least the distal end of the shaft is made from a shape memory alloy.

17. The arthroscopic probe according to claim 13, wherein the probe further comprises a battery for energizing the shape memory alloy to allow a change of the form of the distal end of the shaft.

18. The arthroscopic probe according to any of the preceding claims, wherein the longitudinal probe is disposable.

19. The arthroscopic probe according to claim 15, wherein mandrel is disposable.
